# NEW EUROPEAN PATENT SPECIFICATION

(11) **EP 0 804 129 B2**
(45) Date of publication and mention of the opposition decision: **03.10.2007**
(45) Mention of the grant of the patent: 28.08.2002
(21) Application number: 96902080.9
(22) Date of filing: 11.01.1996
(51) Int. Cl.: A61F 2/16

(54) **IOL INSERTION APPARATUS**
VORRICHTUNG ZUM EINBRINGEN EINER INTRAOKULARLINSE
APPAREIL DESTINE A L'INSERTION D'UNE LENTILLE INTRA-OCULAIRE

(30) Priority: 17.01.1995 US 373822
(43) Date of publication of application: 05.11.1997
(73) Proprietor: Advanced Medical Optics, Inc., Santa Ana, CA 92705 (US)
(72) Inventor: YANG, Shih-Liang, Stanley, Laguna Hills, CA 92653 (US); CUNANAN, Crystal, M., Mission Viejo, CA 92692 (US); McNICHOLS, Thomas, M., Laguna Nigueal, CA 92677 (US)
(74) Representative: Baker, Colin John
(86) International application number: PCT/US1996/000118
(87) International publication number: WO 1996/022062

(56) References cited:
- EP-A- 0 233 708
- EP-A- 0 340 698
- EP-A- 0 480 809
- EP-B- 0 910 311
- WO-A-79/00327
- WO-A-93/16176
- WO-A-94/07436
- WO-A-94/20027
- DE-A- 3 610 925
- US-A- 4 487 865
- US-A- 4 500 676
- US-A- 4 681 102
- US-A- 4 810 586
- US-A- 4 847 324
- US-A- 4 867 126
- US-A- 5 077 352
- US-A- 5 094 876
- US-A- 5 272 012
- US-A- 5 304 182
- US-A- 5 338 312

## Description

### Background of the Invention

The present invention relates to apparatus for inserting an intraocular lens through a small incision into an eye as specified in the preamble of claim 1. Such an apparatus is eg known from WO 94/20027. More particularly, the invention relates to a modified apparatus for inserting a foldable intraocular lens into an eye. Furthermore, covalently bonding lubricating component on outer tissue contacting polymer surfaces of medical devices is in general known from US-A-5 094 876.

An intraocular lens (IOL) is implanted in the eye, for example, as a replacement for the natural crystalline lens after cataract surgery or to alter the optical properties of (provide vision correction to) an eye in which the natural lens remains. IOLs often include an optic, and preferably at least one flexible fixation member or haptic which extends from the optic and becomes affixed in the eye to secure the lens in position. The optic normally includes an optically clear lens. Implantation of such IOLs into the eye involves making an incision in the eye. It is advantageous, to reduce trauma and speed healing, to have an incision size as small as possible.

IOLs are known which are foldable (deformable) so that the IOL can be inserted through a smaller incision into the eye. A substantial number of instruments have been proposed to aid in inserting such a foldable lens in the eye.

Many of the prior art IOL insertion systems load and/or fold the lens at the distal end, that is at the end closest to the eye or the end inserted into the eye. Such "distal loading" systems often disadvantageously include a space consuming loading component at or near the distal end of the system which causes the distal end to be relatively large. This relatively large distal end makes inserting the IOL through a small incision more difficult, if not impossible. Systems which fold and load the IOL proximally of the distal end provide certain advantages, such as reduced stress on the IOL and/or inserter, relative to "distal loading" systems.

However, whether using a distal loading or proximal loading system, one factor which limits the size of the inserter tube involves the inserter tube itself. For example, the material from which the inserter tube is made, for example, polypropylene and the like polymeric materials, may not be compatible or otherwise susceptible to causing optics, for example, made from silicone polymeric materials, to pass through relatively small hollow spaces. For example, the injector tubes may be made of materials, in particular polymeric materials, which are relatively hydrophobic and/or have insufficient lubricity to facilitate the passage of a folded IOL through the tube.

As a result of this incompatibility or lack of lubricity, the hollow space of the injector tube must be made relatively larger to accommodate the folded intraocular lens. This is detriment since, as noted above, it is advantageous to have the smallest possible incision for insertion of the IOL. In addition, if one were to use a small diameter tube to pass the IOL, excessive force might be needed to pass the IOL through the small hollow space thereby increasing the risks of damaging the IOL and, in extreme cases, even damaging the eye into which the IOL is placed.

One approach that may be considered is to use a lubricity agent in passing the IOL through the insertion apparatus. However, such lubricity agents in and of themselves occupy valuable space, thereby at least partially defeating the purpose of using such agents. Also, such lubricity agents often end up in the eye, thereby creating the risk of causing trauma and/or irritation and/or damage to the eye.

It would be advantageous to provide an IOL insertion apparatus and method for using same which facilitates the passage of a folded IOL through the apparatus in a controlled manner without using excessive force.

WO 94/20027 discloses the use of visco-elastic solutions and/or balanced salt solutions to lubricate the passage of an IOL through an inserter.

US 5,094,876 discloses lubricating the outer tissue contacting surfaces of medical devices to improve the tissue contacting characteristics of such surfaces.

### Summary of the Invention

New apparatus for injecting IOLs and methods for using such apparatus have been discovered. The present apparatus achieve enhanced lubricity without contaminating the eye, thus providing for the use of effective reliable and non-excessive amounts of force to inject a folded IOL into an eye. The present invention is straightforward, easy to practice, and involves little or no modification of surgical techniques. In other words, no separate or different lubricity agents are required during the procedure in which the IOL is inserted into the eye. Further, no additional agents are provided to the eye, thereby reducing the risks of contamination, irritation and post-operative trauma. The methods according to which the device of the present invention can be used are straightforward and easy to practice, and often involve surgical techniques which are well practiced and conventionally used to insert IOLs into eyes..

In general, the present invention involves apparatus for inserting IOLs into an eye which include a lubricity enhancing component covalently bonded to the apparatus, to the interior wall defining a hollow space through which an IOL is passed, to facilitate the passage of the IOLs through the apparatus. Covalent attachment or bonding of such lubricity enhancing components is particularly effective since the amount of such component present, and therefore the degree of enhanced lubricity, is conveniently controlled and stable on a long term basis, for example, has a long term shelf life. In addition, there is reduced chance or risk of the lubricity enhancing component being disadvantageously removed from the surface of the apparatus as the IOL passes through the apparatus into the eye.

The use of the present covalently bonded lubricity enhancing components allows successful injection of silicone-based IOLs, for example, employing inserters made of polypropylene and the like polymeric materials, through incisions about 3.2 mm or less, preferably about 3.0 mm or less, and still more preferably about 2.8 mm or less.

In one broad aspect of the present invention, apparatus for inserting an IOL through a small incision into the eye are provided. Such apparatus comprise a hollow tube including an interior wall defining a hollow space through which an IOL is passed and an outlet or end opening through which the IOL is passed from the hollow space into an eye. A lubricity enhancing component is covalently bonded to the hollow tube at the interior wall in an amount effective to facilitate the passage of the IOL through the hollow space. The lubricity enhancing component is covalently bonded to the hollow tube using methods known in the art, such as plasma and/or other activation of the tube to form functional groups that are chemically bondable to functional groups on the precursors to the lubricity enhancing components, and employing a difunctional linking component which is effective to react with both the tube and the lubricity enhancing component precursor. Other methods may be employed to covalently bond the lubricity enhancing component to the hollow tube.

Thus, the lubricity enhancing component is effective to reduce the force needed to pass the IOL through the hollow space of the tube relative to the force needed to pass an identical IOL through the hollow space of a similar apparatus without the lubricity enhancing component. This "reduced force" feature of the present invention is particularly useful, even when no reduction in the size of the incision is obtained. The use of reduced force allows the surgeon to have more control of the rate at which the IOL is inserted into the eye and, in addition, reduces the risk of damage to the eye during IOL insertion.

The hollow tube is preferably made of a polymeric material, more preferably selected from polypropylene and the like materials.

The lubricity enhancing component is preferably selected from the group consisting of hydrophilic components, oleophilic components and mixtures thereof.

In one useful embodiment, the present apparatus further comprises a loading portion sized and adapted to receive an IOL for passage into the hollow space. The lubricity enhancing component is preferably covalently bonded to the loading portion in an amount effective to facilitate the passage of the IOL into the hollow space. Thus, both the hollow tube and the loading portion include effective amounts of the lubricity enhancing component to facilitate passage of the IOL from the loading portion into the hollow tube and from the hollow tube into the eye. In addition, it is more convenient to treat both the hollow tube and loading portion, which together are preferably a single, integrally formed unit, with the lubricity enhancing component, rather than treating only the hollow tube with such component.

The loading portion is preferably sized and adapted to receive an IOL, for example, in an unfolded state, and to hold the IOL in a folded state. The loading portion can be structured to at least facilitate the folding of the IOL from the unfolded state to a folded state. The hollow tube includes an interior wall which defines a hollow space preferably sized to receive the IOL in a folded state from the loading portion and to pass the folded IOL to an open outlet through which the IOL is passed into an eye.

The apparatus of the invention can be used in methods for inserting an IOL into an eye. Such methods can comprise placing an outlet or end opening of a hollow tube in or in proximity to an incision in an eye, and passing an IOL from the hollow tube through the outlet or opening into the eye. The hollow tube includes an interior wall defining a hollow space through which an IOL in a folded state is passed. A lubricity enhancing component is provided and is covalently bonded to the hollow tube at the interior wall in an amount effective to facilitate passing the IOL in a folded state through the hollow space. In a particularly useful embodiment, the hollow tube is made of a polymeric material, such as polypropylene and the like polymeric materials, and the IOL comprises an optic including a silicone polymeric material.

These and other aspects of the present invention will become apparent in the following detailed description and claims, particularly when considered in conjunction with the accompanying drawings in which like parts bear like reference numerals.

### Brief Description of the Drawings

Fig. 1 is an schematic illustration showing an IOL inserter in accordance with the present invention being treated to form a covalently bonded lubricity enhancing component thereon.

Fig. 2 is a fragmentary illustration of a small portion of the IOL inserter shown in Fig. 1 after having been treated to form a lubricity enhancing component thereon.

Fig. 3 is a front side view, in perspective, of an apparatus in accordance with the present invention with the load chamber in the open position.

Fig. 4 is a side view, in perspective, of the apparatus shown in Fig. 3 with the load chamber in the closed position.

Fig. 5 is a front side view, in perspective, of the apparatus shown in Fig. 4 loaded into a hand piece.

Fig. 6 is a side view, partly in cross-section, taken generally along line 6-6 of Fig. 5.

Fig. 7 is a somewhat schematic illustration showing the apparatus shown in Fig. 5, with the hand piece partially in cross-section, being used to insert an IOL into an eye.

### Detailed Description of the Drawings

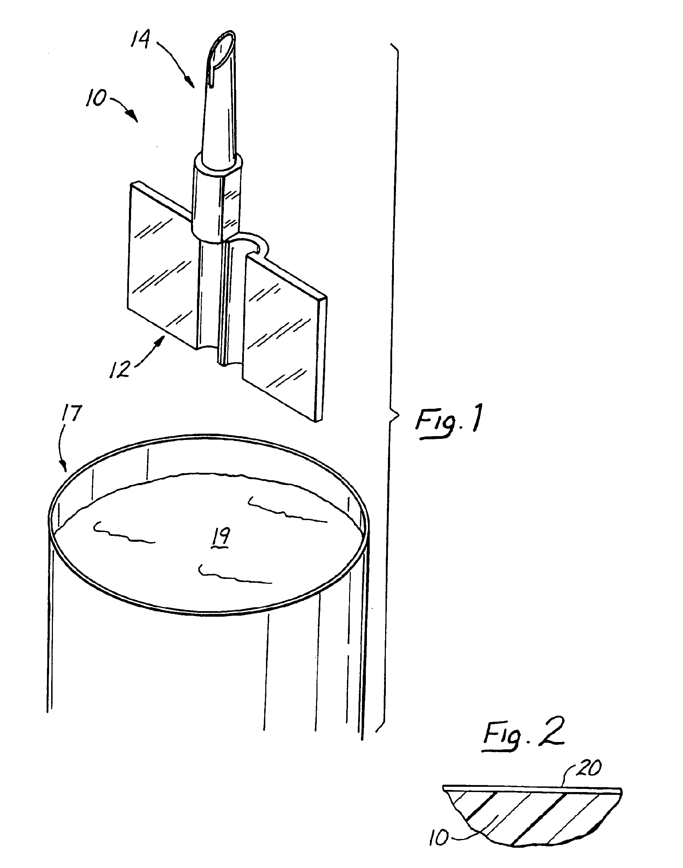

Fig. 1 illustrates an IOL inserter, shown generally at 10, including a load chamber 12 and an injection tube 14. In Fig. 1, the IOL inserter 10, which is made of polypropylene, is shown about to be immersed in a bath 16 of a precursor of a hydrophilic component 19, such as polyethylene glycol. The purpose of treating IOL inserter 10 with the hydrophilic component precursor 19 is to covalently bond all of the exposed surfaces of IOL inserter 10 with a hydrophilic component which enhances the lubricity of IOL inserter 10, for example, of the lumens defined by load chamber 12 and injection tube 14 to facilitate the passage of a folded IOL therethrough.

Forming a covalently bonded hydrophilic component on the exposed surfaces of IOL inserter 10 can be accomplished in any one of a number of ways. Examples of forming such covalently bonded coatings include those set forth in the following: Guire U.S. Patent 4,979,959; Guire U.S. Patent 5,263,992; Guire U.S. Patent 4,722,906; Guire et al U.S. Patent 5,217,492; and Guire et al U.S. Patent 5,258,041.

One useful method of covalently bonding a hydrophilic component, such as polyethylene glycol residue, to IOL inserter 10 is as follows. A quantity of polyethylene glycol of appropriate molecular weight, for example, about 1000, is reacted with a moiety to form a compound having the formula

A - PEG

wherein A is a reactive group, for example, selected from a photochemically reactive group, a thermochemically reactive group or other type of reactive group, capable in response to specific activation of bonding covalently to the surfaces of IOL inserter 10; and PEG is the residue of the original polyethylene glycol employed, and includes substantially all of the hydrophilic properties or characteristics of this original material.

The photochemically reactive groups (A) (the covalent bonding of which is activated by actinic radiation) may be typified by aryl, alkyl and acyl azides, oxazidines, isocyanates (nitrene generators), alkyl and 2-ketodiazo derivatives and diazirines (carbene generators), aromatic ketones (triplet oxygen generators), aromatic diazonium derivatives and numerous classes of carbonium ion and radical generators. Reference is made to Frederick J. Darfler and Andrew M. Tometsko, chapter 2 of Chemistry and Biochemistry of Amino Acids, Peptides and Proteins (Boris Weinstein, ed) vol. 5, Marcel Dekker, Inc. New York, 1978, for further description of photochemically reactive groups.

Thermochemically reactive groups (A) (that are activated by heat energy) are typified by and include nitrophenyl-halides, alkylamino, alkylcarboxyl, alkylthiol, alkylaldehyde, alkylmethylimidate, alkylisocyanate, alkylisothiocyanate, alkylhalide and the like groups.

Groups appropriate for use as thermochemically reactive groups (A) include carboxyl groups, hydroxyl groups, primary amino groups, thiol groups, maleimides, halide and the like groups. N-oxysuccinimide carboxylic esters of such groups as 6-amino hexanoic acid and amino undecanoic acid, alkylthiol groups such as mercaptosuccinic anhydride and beta mercaptopropionic acid, homocysteinethiolactones, and polyethylene glycol derivatives are specific examples.

The compound, that is A - PEG, thus formed is applied to the exposed surfaces of IOL inserter 10, for example, by dipping or immersing the IOL inserter in a solution containing the compound. The resulting coated IOL inserter 10 is then subjected to the specific activation energy, e.g., light and/or heat and/or the like, effective to cause the group A to react with the IOL inserter at conditions effective to covalently bond the residue of the compound, A-PEG, to the IOL inserter. As shown in Fig. 2, IOL inserter 10, after the above-noted processing, includes a hydrophilic coating 20 of the compound A - PEG residue on all surfaces.

In another method of covalently bonding a lubricity enhancing component to an IOL inserter, the IOL inserter is pre-treated, for example, with plasma, to activate the exposed surfaces thereof so that such exposed surfaces are susceptible to being covalently bonded to a lubricating enhancing component. In a particularly useful embodiment, the exposed surfaces of the IOL inserter are subjected to plasma to form functional groups thereon which are effective to covalently bond to a functional group in the lubricity enhancing component precursor. Directly after the IOL inserter is activated to form such functional groups on the exposed surfaces thereof, the IOL inserter is dipped or immersed in the precursor for a period of time so that substantially all of the exposed surfaces of the IOL inserter are completely wetted with the precursor. At this point, thes IOL inserter 10 is removed from the bulk of the precursor and is placed at conditions, for example, exposed to effective light energy of a specific wave length, to facilitate the photo reaction of the lubricity enhancing component precursor with the exposed surfaces, for example, the functional groups on the exposed surfaces, of the IOL inserter. Once this reaction has occurred, the IOL inserter includes a coating of the lubricating enhancing component on all exposed surfaces thereof.

A further method of covalently bonding a lubricity enhancing component to an IOL inserter involves the use of gamma ray and/or electron beam irradiation to induce graft polymerization. In general, an IOL inserter is immersed in a monomer (for example, a compound from which a hydrophilic polymeric component is derived) or a solution containing a monomer. The immersed IOL inserter is then subjected to gamma ray and/or election beam irradiation to induce graft polymerization of the monomer onto the surface of the IOL inserter. The use of gamma ray and/or election beam irradiation to induce graft polymerization is more fully described in Goldberg et al U.S. Patent 4,806,382; Goldberg et al U.S. Patent 5,094,876; Goldberg et al U.S. Patent 5,108,776; Goldberg et al U.S. Patent 5,130,160; and Goldberg et al U.S. Patent 5,290,548. Each of these patents is directed to providing biocompatibility and does not teach or suggest enhancing lubricity or IOL inserters.

The lubricity enhancing component is preferably covalently bonded to the IOL inserter without employing gamma ray or electron beam irradiation. Sources of gamma ray and electron beam irradiation are relatively expensive and bulky so that it is relatively difficult to use such irradiation at the facility where the IOL inserters are produced. In addition, subjecting the IOL inserter immersed in a monomer-containing solution to gamma or electron beam irradiation may result in a graft polymeric coating which is substantially thicker than desired. This can lead to an additional and disadvantageous processing step in which excess coating material is removed. In view of these problems, it is preferred to employ other methods to provide an IOL inserter with a covalently bonded lubricity enhancing component.

The lubricity enhancing components useful in the present invention may be selected from suitable components which function as such lubricity enhancing components as described herein. The lubricity enhancing component is present in an amount effective to enhance the lubricity of the interior wall of the hollow tube defining a hollow space through which the IOL passes in being inserted into the eye. Such lubricity enhancing components are preferably effective to provide such enhanced lubricity for relatively long periods of time, for example, for at least about 1 month or at least about 3 months or at least about 6 months, so that the IOL inserter has a relatively long shelf life and can be used after being packaged and stored for such relatively long periods of time and still obtain the substantial enhanced lubricity benefits.

The covalent bonding of the lubricity enhancing component to the IOL inserter is preferably effective to maintain this component secured to the inserter as the IOL is inserted into the eye. In other words, it is preferred that such covalent bonding is effective to substantially prevent the lubricity enhancing component from passing into the eye as the IOL is inserted into the eye. Thus, the present invention conveniently provides for enhanced lubricity and ease of inserting an IOL into an eye while, at the same time, reducing, or even eliminating, contaminating the eye with lubricant.

Particularly useful lubricity enhancing components include those selected from hydrophilic components, oleophilic components and mixtures thereof. Examples of hydrophilic lubricity enhancing components include, but are not limited to, those derived from polyethylene glycol, polyvinylpyrrolidone, poly (N-vinyl lactams), polyacrylic acid, polyethylene oxide, polypropylene oxide, polyvinyl pyridine, polyvinyl alcohol, polysaccharides, polycarboxyl methyl cellulose, polymethacrylic acid, polyacrylamide, polypeptides, poly sodium styrene sulfonate, polyhydroxyethyl methacrylate, heparin and the like and mixtures thereof. If a hydrophilic lubricity enhancing component is employed, it is preferred that the IOL inserter be immersed or otherwise contacted with water, for example, a saline solution, to hydrate the hydrophilic component. Such hydration is effective to facilitate the lubricity enhancing characteristics of the hydrophilic component.

Lubricity enhancing components which are not significantly hydratable by an isotonic aqueous liquid at room temperature may be considered oleophilic lubricity enhancing components. Examples of useful oleophilic lubricity enhancing components include, but are not limited to, those derived from carboxylic acids having about 10 to about 30, carbon atoms per molecule, glycerol monostearate, glycerol monopalmitate, glycerol monooleate and the like and mixtures thereof.

It should be noted that, because of the covalent bonding involved in the present invention, the lubricity enhancing components identified herein are often present in a slightly altered form relative to the more commonly known precursors of such components. However, such lubricity enhancing components often have substantially the same or better lubricity enhancing properties relative to the corresponding precursors and, in addition, have the added advantage of being covalently bonded to the IOL inserter.

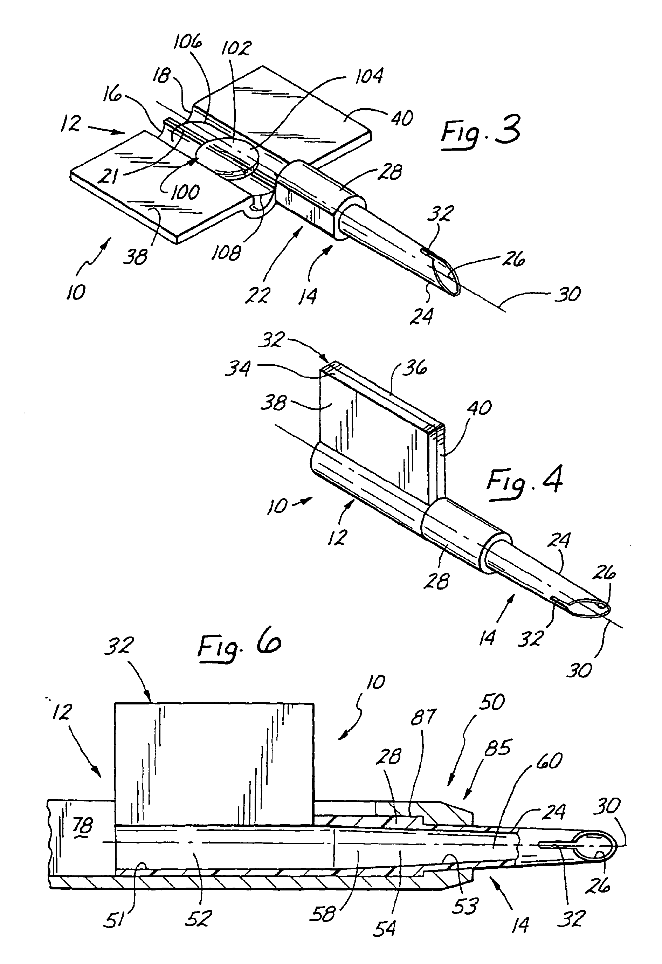

Figs. 3 to 7 illustrate the use of IOL inserter 10 including a coating of lubricity enhancing component 20 on all exposed surfaces thereof.

The body of IOL inserter 10 (that is other than coating 20) is an integrally formed, for example, molded, unit made of propropylene. Load chamber 12 includes a first member 16 and a second member 18 which are secured or joined together and are hingeably moveable relative to each other along line 21, which is parallel to the longitudinal axis 30 of inserter 10.

Injection tube 14 includes a proximal end portion 22, a distal end portion 24 and an open distal end 26. A reinforcing collar 28 is coincidental with the proximal end portion 22 of injection tube 14.

Open distal end 26 is beveled at an angle of about 45° relative to the longitudinal axis 30 of the inserter 10.

Injection tube 14 includes a through slot 32 which extends from the open distal end 26 distally and terminates prior to the proximal end portion 22 of injection tube 14. Through slot 32 is elongated in a direction parallel to the longitudinal axis 30 of inserter 10.

As shown in Fig. 3, inserter 10 is in the opened position. In contrast, in Fig. 4, inserter 10 is shown in the closed position. In the closed position, the load chamber 12 includes a top 32 which is a combination of top surfaces 34 and 36 of first wing 38 and second wing 40, respectively, of first member 16 and second member 18, respectively. First and second wings 38 and 40 are effective for a human user of inserter 10 to hold and manipulate the inserter 10 while using it, as described hereinafter.

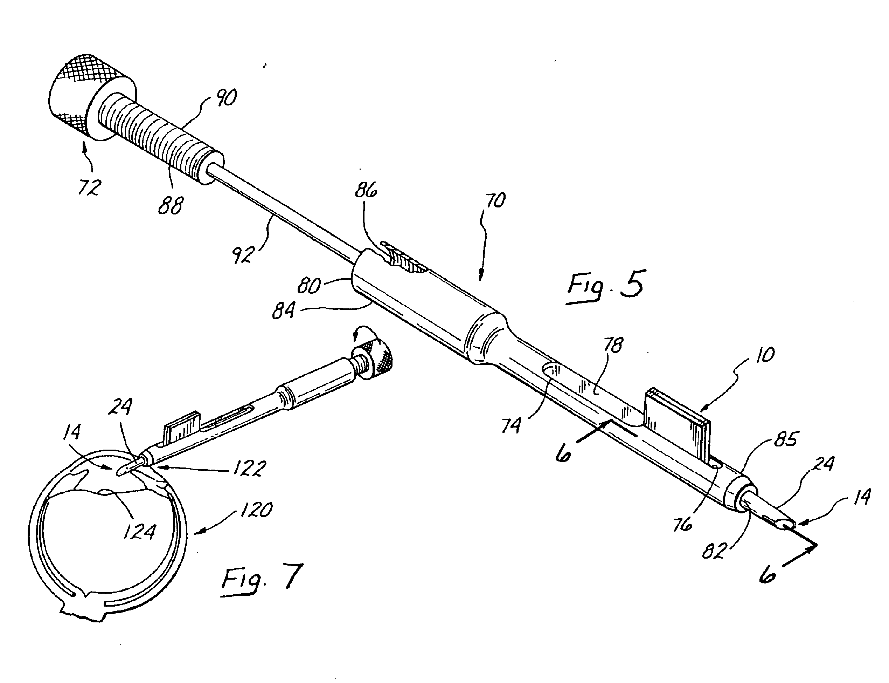

Inserter 10 is described in more detail with reference to Fig. 5, which shows the inserter in combination with hand piece 50. When used in combination with hand piece 50, the load chamber 12 of inserter 10 is in the closed position, as shown in Fig. 4. With the load chamber 12 in the closed position, and top 32 being the uppermost portion of the load chamber, open distal end 26 of injection tube 14 is beveled at an angle of 45° relative to the longitudinal axis 30 of the inserter 10 so that the open distal end is generally right facing (when the inserter is viewed from above). In addition, through slot 32 intersects the open distal end 26 at the proximal most portion of the open distal end, as shown in Figs. 3, 4 and 6.

Referring to Fig. 6, with load chamber 12 in the closed position, the load chamber includes an interior wall 51 which defines a first lumen 52 that is elongated in a direction parallel to the longitudinal axis 30 of inserter 10. Injection tube 14 includes a tapering interior wall 53 which defines a distally tapering second lumen 54. The average cross-sectional area of second lumen 54 transverse to the longitudinal axis 30 is smaller than or reduced relative to the average cross-sectional area of the first lumen 52.

The first lumen 52 is aligned with the second lumen 54 so that a folded IOL in the first lumen can be passed directly from the first lumen into the second lumen. The taper of proximal portion 58 of second lumen 54 is more severe than the slight taper which exists in the distal portion 60 of the second lumen. The more severe taper in the proximal portion 58 is effective to further fold the IOL as the IOL is passed into the second lumen 54. This further folding is advantageous because the further folded IOL can be inserted into the eye through a smaller incision. The enhanced lubricity resulting from the coating 20 facilitates this further folding so that a reduced amount of force is required to further fold the IOL and/or the degree of further holding of the IOL can be increased so that ultimately, the IOL can be inserted through an even smaller incision. The coating 20 also advantageously reduces the risk of tearing and/or otherwise damaging the IOL as the IOL is passed through the first lumen 52 and second lumen 54.

With reference to Fig. 5, inserter 10 is shown in combination with hand piece 70 and push rod member 72. Hand piece 70 includes a relatively large, elongated first through opening 74 and a relatively small, elongated second through opening 76. Hand piece 70 includes a through bore 78 which extends from the proximal end 80 to the distal end 82 of the hand piece. The proximal end portion 84 of hand piece 70 includes threads 86 which are adapted to engage and mate with threads 88 of the proximal segment 90 of push rod member 72. Rod element 92 of push rod member 72 is adapted to pass through bore 78, first lumen 52, second lumen 54 and out of open distal end 26. Hand piece 70 and push rod member 72 are made of metal, such as surgical grade stainless steel or the like metals.

Inserter 10 is operated and functions as follows. When it is desired to load an IOL into inserter 10, the inserter is placed, for example, manually placed, in a configuration as shown in Fig. 3. With load chamber 12 in the opened position, an IOL, such as is shown generally at 100, is placed, for example, using forceps, in between first and second members 16 and 18. This placement is such that the anterior face 102 of optic 104 faces upwardly, as shown in Fig. 3. The optic 104 is made of a silicone polymeric material. The filament haptics 106 and 108 of IOL 100 are located as shown in Fig. 3, so that the fixation members are located generally parallel to, rather than transverse to, the longitudinal axis 30.

With IOL 100 placed as shown in Fig. 3, first and second members 16 and 18 are hingeably moved relative to each other, for example, by manually bringing first and second wings 38 and 40 together, to place the load chamber 12 in the closed position, as shown in Fig. 4. With load chamber 12 in the closed position, IOL 100 is in a folded state, that is optic 104 is folded. The relative movement of first and second members 16 and 18 to move the load chamber from the open position to the closed position is effective to fold the lens. The folded IOL 100 is now located in the first lumen 52. For clarity sake, the folded IOL is not shown in any of Figs. 4, 5, 6 or 7.

With the inserter 10 configured as shown in Fig. 4 and folded IOL 100 located in first lumen 52, the inserter 10 is placed in association with hand piece 70, as shown in Fig. 5. In this configuration, the distal end portion 24 of injection tube 14 extends distally beyond the distal end 82 of hand piece 70. As shown in Fig. 6, the distal portion 85 of hand piece 70 includes an inner wall 87 which is configured to receive reinforcing collar 28 in abutting relation.

With inserter 10 so placed relative to hand piece 70, push rod member 72 is placed into the through bore 78 of the hand piece starting at the proximal end 80. As threads 88 come in contact with and engage threads 86, the push rod member 72 is rotated, as shown in Fig. 7, so as to thread the push rod member onto the proximal end portion 84 of hand piece 70. By gradually moving push rod element 92 through bore 78 of hand piece 70, the folded IOL 100 is urged to move from first lumen 52 into second lumen 56, through open distal end 26 and into the eye.

Referring now to Fig. 7, the IOL 100 is to be placed in eye 120 into an area formerly occupied by the natural lens of the eye. Fig. 7 shows the sclera 122 having an incision through which the distal end portion 24 of injection tube 14 is passed. Alternately, the incision can be made through the cornea. Distal end portion 24 has a sufficiently small cross-section to pass into the eye 122 through a 3.0 mm incision in the sclera 122.

The injection tube 14 is manipulated within eye 122 until it is positioned so that IOL 100 can be properly positioned in eye 122, that is in the anterior chamber, the posterior chamber, the capsular bag 124 or in the sulcus, after being released. Thus, the surgeon is able to controllably position the distal end portion 24 of injection tube 14, with IOL 100 in the first lumen 52 of load chamber 12. Once distal end portion 24 is so positioned, the rod element 92 is urged distally, by rotating (threading) push rod member 72 onto hand piece 70, to pass the IOL 100 into and through the second lumen 54, through the open distal end 26 of injection tube 14 and into the eye 120. The anterior face 102 of IOL 100 faces generally forwardly in the eye 120 as the IOL is released from the inserter 10. In other words, the IOL 100 passes through first lumen 52, second lumen 54 and open distal end 26 and into eye 120 without flipping or otherwise becoming mispositioned. Only a relatively small amount of, if any, post-insertion re-positioning is needed to properly position IOL 100 in eye 120.

After the IOL 100 has been inserted into the eye, the rod element 92 is moved proximally into the injection tube 14 and the distal end portion 24 of the injection tube is removed from the eye. If needed, the IOL 100 can be re-positioned in the eye by a small, bent needle or similar tool inserted into the same incision.

Once the IOL 100 is properly positioned in eye 120 and inserter 10 is withdrawn from the eye, the incision in the sclera may be mended, for example, using conventional techniques. After use, inserter 10 is preferably disposed of. Hand piece 70 and push rod member 72 can be reused, after sterilization/disinfection.

The following non-limiting examples illustrate certain aspects of the present invention.

### EXAMPLE 1

Three (3) cartridges, having a structure similar to that of the body of IOL inserter 10, made of PD701NW polypropylene (Himont USA Inc.) were treated with plasma (PS0350, Plasmal Science) at 100 watts, in oxygen at a pressure of 6.7 Pa (0.05 torr) for 10 minutes. The treated cartridges were then immersed in a solution of isopropyl alcohol (100 ml), acrylamide (10 grams), n-vinyl pyrrolidone (10 grams) and benzophenone (2 grams) for 3 minutes. The cartridges were then removed and exposed to UV radiation at 10 milliwatts (medium pressure Hg-arc lamp) for 2 minutes. The performance of the cartridges was determined by the number of three piece IOLs (such as an IOL sold under the trademark SI-30NB by Allergan Medical Optics) could be passed through the cartridge before either the cartridge or an IOL broke. As a comparison, three (3) untreated, similarly structured cartridges were also tested.

Results of these tests were as follows:

| Cartridges | IOLs passed through Treated Cartridges | IOLs passed through Untreated Cartridges |
|---|---|---|
| A | 4 | 0 |
| B | 3 | 0 |
| C | 3 | 1 |

### EXAMPLE 2

Three (3) additional cartridges, having a structure similar to that of the body of IOL inserter 10, made of PD701NW polypropylene (Himont USA Inc.) were treated with plasma (PS0350, Plasmal Science) at 50 watts, in oxygen at a pressure of 6.7 Pa (0.05 torr) for 5 minutes. The treated cartridges were then immersed in a solution of tetrahydrofuran (100ml), acrylamide (10 grams), n-vinyl pyrrolidone (10 grams) and azobisisobutyronitrile for 5 minutes. The cartridges were then removed and placed in an oven set at 80°C for 20 minutes. The excess reagents were rinsed off using IPA for 10 minutes in an ultrasonic bath. The performance of the cartridges was determined by the number of three piece IOLs (such as an IOL sold under the trademark SI-30NB by Allergan Medical Optics) could be passed through the cartridge before either the cartridge or an IOL broke. As a comparison, three (3) untreated, similarly structured cartridges were also tested.

Results of these tests were as follows:

| Cartridges | IOLs passed through Treated Cartridges | IOLs passed through Untreated Cartridges |
|---|---|---|
| D | 4 | 0 |
| E | 2 | 1 |
| F | 3 | 0 |

The results of Examples 1 and 2 demonstrate that the treated cartridges in accordance with the present invention enhance the ability of the cartridge to pass IOLs. The enhanced lubricity of these treated cartridges allows reduced force to be used to pass an IOL, relative to the force needed to pass an IOL through an untreated cartridge. This reduced force requirement results in advantageously reducing the risk of harming the cartridge (inserter) and/or the IOL by passing an IOL through the cartridge.

While this invention has been described with respect of various specific examples and embodiments, it is to be understood that the invention is not limited thereto and that it can be variously practiced within the scope of the following claims.

## Claims

1. An apparatus (10) for inserting an intraocular lens (100) through a small incision into an eye comprising:
a hollow injection tube (14) including a tapering interior wall (53) defining a hollow space (54) through which an intraocular lens (100) is passed and an outlet (26) through which said intraocular lens is passed from said hollow space into an eye; wherein
a lubricity enhancing component (20) is covalently bonded to said hollow injection tube (14) at said interior wall (53) in an amount effective to facilitate the passage of said intraocular lens (100) through said hollow space (54), and wherein the hollow space (54) has a proximal portion (58) with a taper which is more severe than the slight taper in distal portion (60) of the hollow space.

2. The apparatus (10) of claim 1 wherein said hollow tube (14) is made of a polymeric material.

3. The apparatus (10) of any of the preceding claims wherein said hollow tube (14) is made of polypropylene.

4. The apparatus (10) of any of the preceding claims wherein said lubricity enhancing component (20) is selected from the group consisting of hydrophilic components, oleophilic components and mixtures thereof.

5. The apparatus (10) of any of the preceding claims wherein said hollow tube (14) is sized to pass said intraocular lens (100) into the eye through an incision no larger than 3.2 mm.

6. The apparatus (10) of any of claims 1 to 4 wherein said hollow tube (14) is sized to pass said intraocular lens (100) into the eye through an incision no larger than 2.8 mm.

7. The apparatus (10) of any of the preceding claims which further comprises a loading portion (12) sized and adapted to receive an intraocular lens (100) for passage into said hollow space (54).

8. The apparatus (10) of claim 7 wherein said lubricity enhancing component (20) is covalently bonded to said loading portion (12) in an amount effective to facilitate the passage of said intraocular lens (100) into said hollow space (54).

9. The apparatus (10) of any of claims 7 and 8 wherein hollow tube (14) is sized to pass said intraocular lens (100) into the eye through an incision no larger than 3.0 mm.

10. The apparatus (10) of any of claims 7 to 9 wherein said loading portion (12) is sized and adapted to fold an intraocular lens into a folded state.

11. A method of manufacturing an apparatus as defined in any one of the preceding claims, which method comprises covalently bonding the lubricity enhancing component (20) to the hollow tube(14) at its interior wall (53) in an amount effective to facilitate the passage of said intraocular lens (100) through said hollow space (54).

12. A method according to claim 11 including the step of subjecting the hollow tube (14) to plasma activation to form functional groups that are chemically bondable to functional groups on a precursor to the lubricity enhancing component.

## Patentansprüche

1. Vorrichtung (10) zur Einfügung einer Intraokularlinse (100) durch einen kleinen Einschnitt in ein Auge, die Folgendes umfasst:
ein Einführungs-Hohlrohr (14), das eine sich verjüngende Innenwand (53), die einen Hohlraum (54) begrenzt, durch den eine Intraokularlinse (100) hindurchgeführt wird, und einen Auslass (26) einschließt, durch den die Intraokularlinse aus dem Hohlraum in ein Auge eingeführt wird; wobei
ein die Gleitfähigkeit erhöhender Bestandteil (20) kovalent an der Innenwand (53) an das Einführungs-Hohlrohr (14) in einer Menge gebunden ist, die zur Erleichterung der Durchführung der Intraokularlinse (100) durch den Hohlraum (54) wirksam ist, und wobei der Hohlraum (54) einen proximalen Teil (58) mit einer Verjüngung aufweist, die stärker ist, als die schwache Verjüngung in dem distalen Teil (60) des Hohlraums.

2. Vorrichtung (10) nach Anspruch 1, wobei das Hohlrohr (14) aus einem Polymermaterial hergestellt ist.

3. Vorrichtung (10) nach einem der vorhergehenden Ansprüche, wobei das Hohlrohr (14) aus Polypropylen hergestellt ist.

4. Vorrichtung (10) nach einem der vorhergehenden Ansprüche, wobei der die Gleitfähigkeit erhöhende Bestandteil (20) aus der Gruppe ausgewählt ist, die aus hydrophilen Bestandteilen, oleophilen Bestandteilen und Gemischen hiervon besteht.

5. Vorrichtung (10) nach einem der vorhergehenden Ansprüche, wobei das Hohlrohr (14) so bemessen ist, dass die Intraokularlinse (100) durch einen Einschnitt von nicht mehr als 3,2 mm in das Auge einführbar ist.

6. Vorrichtung (10) nach einem der Ansprüche 1 bis 4, wobei das Hohlrohr (14) so bemessen ist, dass die Intraokularlinse (100) durch einen Einschnitt von nicht mehr als 2,8 mm in das Auge einführbar ist.

7. Vorrichtung (10) nach einem der vorhergehenden Ansprüche, die weiterhin ein Zuführteil (12) umfasst, das zur Aufnahme einer Intraokularlinse (100) zur Einführung in den Hohlraum (54) bemessen und angepasst ist.

8. Vorrichtung (10) nach Anspruch 7, wobei der die Gleitfähigkeit erhöhende Bestandteil (20) in einer derartigen Menge an das Zuführteil (12) kovalent gebunden ist, dass die Einführung der Intraokularlinse (100) in den Hohlraum (54) erleichtert wird.

9. Vorrichtung (10) nach einem der Ansprüche 7 und 8, wobei das Hohlrohr (14) so bemessen ist, dass die Intraokularlinse (100) durch einen Einschnitt von nicht mehr als 3,0 mm in das Auge einführbar ist.

10. Vorrichtung (10) nach einem der Ansprüche 7 bis 9, wobei das Zuführteil (12) zur Faltung einer Intraokularlinse in einen gefalteten Zustand bemessen und angepasst ist.

11. Verfahren zur Herstellung einer wie in einem der vorhergehenden Ansprüche definierten Vorrichtung, wobei das Verfahren eine kovalente Bindung des die Gleitfähigkeit erhöhenden Bestandteils (20) an das Hohlrohr (14) an dessen Innenwand (53) in einer Menge umfasst, die zur Erleichterung der Durchführung der Intraokularlinse (100) durch den Hohlraum (54) wirksam ist.

12. Verfahren nach Anspruch 11, das den Schritt einschließt, das Hohlrohr (14) zur Bildung funktioneller Gruppen einer Plasmaaktivierung zu unterwerfen, die chemisch an funktionelle Gruppen an einem Vorläufer für den die Gleitfähigkeit erhöhenden Bestandteil bindbar sind.

## Revendications

1. Appareil (10) pour insérer une lentille intraoculaire (100) à travers une petite incision dans un oeil, comprenant :
un tube d'injection creux (14) englobant une paroi interne (53) conique définissant un espace creux (54) à travers lequel on fait passer une lentille intraoculaire (100) et une sortie (26) à travers laquelle on fait passer ladite lentille intraoculaire à partir dudit espace creux dans un oeil, dans lequel
un composant (20) augmentant la lubricité est lié de manière covalente audit tube d'injection creux (14) contre ladite paroi interne (53) en une quantité efficace pour faciliter le passage de ladite lentille intraoculaire (100) à travers ledit espace creux (54) et dans lequel l'espace creux (54) a une partie (58) proximale pourvue d'une conicité plus importante que la légère conicité de la partie distale (60) de l'espace creux.

2. Appareil (10) selon la revendication 1, dans lequel ledit tube creux (14) est constitué d'une matière polymère.

3. Appareil (10) selon l'une quelconque des revendications précédentes, dans lequel ledit tube creux (14) est réalisé en polypropylène.

4. Appareil (10) selon l'une quelconque des revendications précédentes, dans lequel ledit composant (20) augmentant la lubricité est choisi parmi le groupe constitué par des composants hydrophiles, des composants oléophiles et leurs mélanges.

5. Appareil (10) selon l'une quelconque des revendications précédentes, dans lequel ledit tube creux (14) est dimensionné pour faire passer ladite lentille intraoculaire (100) dans l'oeil à travers une incision dont la dimension n'est pas supérieure à 3,2 mm.

6. Appareil (10) selon l'une quelconque des revendications 1 à 4, dans lequel ledit tube creux (14) est dimensionné pour faire passer ladite lentille intraoculaire (100) dans l'oeil à travers une incision dont la dimension n'est pas supérieure à 2,8 mm.

7. Appareil (10) selon l'une quelconque des revendications précédentes, qui comprend en outre une portion de chargement (12) dimensionnée et conçue pour recevoir une lentille intraoculaire (100) à des fins de passage dans ledit espace creux (54).

8. Appareil (10) selon la revendication 7, dans lequel ledit composant (20) augmentant la lubricité est lié de manière covalente à ladite portion de chargement (12) en une quantité efficace pour faciliter le passage de ladite lentille intraoculaire (100) dans ledit espace creux (54).

9. Appareil (10) selon l'une quelconque des revendications 7 et 8, dans lequel ledit tube creux (14) est dimensionné pour faire passer ladite lentille intraoculaire (100) dans l'oeil à travers une incision dont la dimension n'est pas supérieure à 3,0 mm.

10. Appareil (10) selon l'une quelconque des revendications 7 à 9, dans lequel ladite portion de chargement (12) est dimensionnée et conçue pour amener une lentille intraoculaire à l'état replié.

11. Procédé de fabrication d'un appareil tel que défini dans l'une quelconque des revendications précédentes, ledit procédé comprenant le fait de lier de manière covalente le composant (20) augmentant la lubricité au tube creux (14) contre sa paroi interne (53) en une quantité efficace pour faciliter le passage de ladite lentille intraoculaire (100) dans ledit espace creux (54).

12. Procédé selon la revendication 11, englobant l'étape consistant à soumettre le tube creux (14) à une activation au plasma pour former des groupes fonctionnels qui peuvent être liés par voie chimique à des groupes fonctionnels sur un précurseur du composant augmentant la lubricité.
